# EUROPEAN PATENT SPECIFICATION

(11) **EP 2 328 610 B1**
(45) Date of publication and mention of the grant of the patent: **18.03.2015**
(21) Application number: 09785017.6
(22) Date of filing: 26.08.2009
(51) Int. Cl.: A61K 39/00

(54) **NEMATODE VACCINE**
NEMATODEN-IMPFSTOFF
VACCIN CONTRE LES NÉMATODES

(30) Priority: 28.08.2008 GB 0815674
(43) Date of publication of application: 08.06.2011
(73) Proprietor: Moredun Research Institute, Bush Loan Penicuik EH26 0PZ (GB)
(72) Inventor: SMITH, William, David, Penicuik EH26 0PZ (GB); NEWLANDS, George, Fredrick, James, Penicuik EH26 0PZ (GB); EKOJA, Susan, Waltham Grimsby North East Lincolnshire DN37 0YE (GB); CACHAT, Elise, Penicuik EH26 0PZ (GB)
(74) Representative: Chapman, Paul Gilmour
(86) International application number: PCT/GB2009/002083
(87) International publication number: WO 2010/023452

(56) References cited:
- WO-A-94/19010
- US-A- 5 650 154
- US-A- 5 747 046
- TAVERNOR ANGELA S ET AL: "Immune response of Clun Forest sheep to vaccination with membrane glycoproteins from Haemonchus contortus" PARASITE IMMUNOLOGY (OXFORD), vol. 14, no. 6, 1992, pages 671-675, XP009125030 ISSN: 0141-9838
- LEJAMBRE L F ET AL: "Vaccination against Haemonchus contortus: Performance of native parasite gut membrane glycoproteins in Merino lambs grazing contaminated pasture" VETERINARY PARASITOLOGY, ELSEVIER SCIENCE, AMSTERDAM, NL, vol. 153, no. 3-4, 31 May 2008 (2008-05-31), pages 302-312, XP022621226 ISSN: 0304-4017 [retrieved on 2008-02-03]
- KNOX DAVID P ET AL: "The nature and prospects for gut membrane proteins as vaccine candidates for Haemonchus contortus and other ruminant trichostrongyloids." INTERNATIONAL JOURNAL FOR PARASITOLOGY, vol. 33, no. 11, 2003, pages 1129-1137, XP002553497 ISSN: 0020-7519
- SMITH W D ET AL: "Preliminary observations on the potential of gut membrane proteins of Haemonchus contortus as candidate vaccine antigens in sheep on naturally infected pasture" VETERINARY PARASITOLOGY, vol. 98, no. 4, 27 July 2001 (2001-07-27), pages 285-297, XP002553498 ISSN: 0304-4017
- SMITH W D ET AL: "Cross-protection studies with gut membrane glycoprotein antigens from Haemonchus contortus and Teladorsagia circumcincta" PARASITE IMMUNOLOGY (OXFORD), vol. 23, no. 4, April 2001 (2001-04), pages 203-211, XP002553499 ISSN: 0141-9838

## Description

### Field of the Invention

The present invention relates to purified antigens derived from nematodes, such as *Haemonchus contortus* and their use in low doses to vaccinate susceptible animals. There is also provided a vaccine comprising a low dose of a purified nematode antigen such as an *Haemonchus contortus* antigen, as well as methods and dosage regimes for vaccinating/immunising susceptible animals.

### Background to the Invention

*Haemonchus contortus* is the single most important nematode parasite of sheep and goats in the world. Since it prefers warm moist climates, *Haemonchus* is much commoner in the tropics and sub-tropics than in temperate zones. The parasite inhabits the true stomach or abomasum where the late larval and adult stages feed on blood. It is capable of causing substantial blood loss, so that the host becomes anaemic and may die.

Control has relied on anthelmintic drugs combined with pasture management. However drug resistant Haemonchus are becoming increasingly widespread and the need to find alternative control methods is pressing. Vaccination is one potential solution but to date there are no commercial vaccines for any gut nematode parasite of any host.

The prospects for a Haemonchus vaccine are much better than for any other nematode species because numerous published trials, including a few done under field conditions, have shown that substantial protection can be achieved by immunising sheep with antigens extracted from the worm's intestinal cells (Reviewed by Knox and Smith 2001; Knox, et al. 2003; Newton and Meeusen 2003; Smith and Zarlenga 2006).

Vaccination with these antigens generates circulating host antibodies. When the parasite feeds it ingests these antibodies with its blood meal. The antibodies bind to and disrupt the function of the parasite intestine leading to substantially reduced egg counts and smaller worm burdens.

Various antigens have been isolated from the intestinal cells of Haemonchus and shown to be protective when tested in vaccine trials in sheep. However only two of these antigens, namely H-gal-GP (WO94/02169) and H11 (WO88/00835), have consistently conferred protection to a degree (>80% efficacy in >80% of the flock) which would exceed a conventional anthelmintic programme (Barnes, Dobson and Barger 1995) and which would therefore be commercially useful. Native H-gal-GP and H11 have each been shown to reduce Haemonchus egg counts by more than 90% in vaccinated sheep and, when used in combination, their effect in a controlled field trial was highly beneficial for grazing Merino lambs, preventing deaths, reducing anaemia and substantially preventing transmission of infective larvae (Le Jambre, Windon and Smith, 2008).

Routinely, H-gal-GP and H11 are isolated simultaneously from the same detergent extract of Haemonchus membranes by lectin affinity chromatography using peanut and ConA agarose columns connected in sequence but then eluted separately (Smith et al 2000). The peanut binding fraction is almost exclusively H-gal-GP. The ConA binding fraction is enriched for the family of aminopeptidases known as H11 (Newton and Meeusen 1993), but also contains other less abundant components including so called "P1" (Smith et al 1993), GA1 (Jasmer et al 1993) and some H-gal-GP. Approximately 0.5mg of each fraction can be extracted from 1g of adult Haemonchus parasites.

At least two doses of H11 or H-gal-GP are needed to stimulate protection. Early vaccine trials with the native H11 fraction mixed with aluminium hydroxide and Freund's as adjuvant, indicated that it was not consistently protective for sheep when used at less than 50ug protein per dose and it was assumed that the protective dose of native H-gal-GP would be in the same order. However, a maximum of 5-10 g of adult Haemonchus can be harvested from a deliberately infected sheep. Hence the maximum yield of native H-gal-GP (or H11) from a donor sheep is about 5mg and it was assumed that this represented only 100 doses of vaccine. When the labour cost of worm harvesting and antigen extraction are added to the sheep worm donor costs, it can be seen that preparing a native antigen vaccine in this way could never be commercially viable.

Not surprisingly, a lot of work was then done to produce protective recombinant forms of H-gal-GP (Newlands et al 2006; Smith et al 2003 a and b), H11 (Newton and Meeusen 1993) or other potentially protective Haemonchus proteins (see for example WO08/032073) to create a commercial vaccine. However, much work remains unpublished probably because no useful protection was obtained. The very fact that much more time and money has been invested in the recombinant, rather than the native antigen approach suggests that no one believed the latter was commercially feasible.

H-gal-GP is an intestinal cell brush border complex. The protective components consist of a family of four metalloproteases combined with a family of two aspartyl proteases (Newlands et al 2006; Smith et al 2003 a and b). The native complex loses most of its protective activity if it is dissociated, which indicates that conformational epitopes are important and explains why bacterially expressed insoluble recombinants do not work (Smith et al 2003 a and b). Recently, most of the H-gal-GP enzymes were expressed in eukaryotic systems as soluble proteins, but when a cocktail of these was evaluated in a sheep vaccine trial, no protection was conferred.

The 3-D structure of H-gal-GP complex has recently been visualised by electron microscopy (Meunch et al, 2008). The complex's shape is unique and intriguing particularly as it contains an internal chamber with three openings. Since vaguely analogous complexes which function as protease "machines" have been described in bacteria (Tamura et al 1996; Franzetti et al 2002), it is hypothesised that the structure of the complex has evolved to efficiently dock and then digest protein substrates in the parasite's blood meal. Although more work is needed to elucidate the stoichiometry of the complex, its quaternary structure goes a long way to explain why a cocktail of recombinant versions of its individual sub-units do not protect.

This last result suggested that the recombinant antigen approach was unlikely to be successful for H-gal-GP.

It is amongst the objects of the present invention to obviate and/or mitigate at least one of the aforementioned disadvantages.

### Summary of the invention

The present invention is based on observations by the present inventors that a surprisingly low dose of purified native antigen is capable of raising a protective immune response in an animal.

An immunogenic formulation for use in protecting and/or treating an animal against a parasitic nematode, wherein the immunogenic formulation comprises a purified integral intestinal cell membrane glycoprotein antigen from a nematode obtained from an infected animal wherein the intestinal cell membrane associated antigen is H-gal-GP and/or H11 antigen, or species specific variant, mutant or homologue thereof in an amount between 1µg to 10µg. Typically the amount of purified antigen will be less than or equal 5µg, 4µg, 3µg, or 2µg.

In a further aspect there is provided a process for preparing an immunogenic formulation for use in a method for protecting and/or treating an animal against a parasitic nematode, which process comprises:
a) isolating nematodes from an infected animal;
b) obtaining membranous material from said nematodes;
c) purifying a membrane associated antigen from said membranous material, wherein the intestinal cell membrane associated antigen is H-gal-GP and/or H11 antigen, or species specific mutant displaying similar functional activity thereof; and
d) preparing said formulation comprising between 1µg and 10µg of said purified antigen.

Typically this may be carried out as exemplified in Smith et al 2001 , or as described in pending patent application WO2010023452 - filed 3rd August 2009.

Preferred amounts of antigen are as hereinbefore described.

The immunogenic formulation may be prepared by mixing, preferably homogeneously mixing, said antigen of the invention with a pharmaceutically and/or veterinarally acceptable carrier, diluent, excipient and/or adjuvant using standard methods of pharmaceutical and/or veterinary preparation.

In a further aspect there is provided use of a purified native parasitic nematode integral membrane antigen H-gal-GP and/or H11 antigen or specific variant, mutant or homologue thereof in the manufacture of a medicament for vaccinating or immunising a susceptible animal against said parasitic nematode wherein the medicament is intended to be administered in a dose of between 1µg and 10µg per animal per dose.

In a further aspect there is provided a formulation comprising between 1µg and 10µg of purified membrane associated H-gal-GP and/or H11 antigen or specific variant, mutant or homologue thereof from a parasitic nematode for use in vaccinating a susceptible animal.

Preferred doses are as hereinbefore described.

In a further aspect there is provided a method of vaccinating a susceptible animal against infection from a parasitic nematode, said method comprising the step of administering between 1µg to 10µg of purified H-gal-GP and/or H11 antigen or specific variant, mutant or homologue thereof to said animal in order to raise a suitable immune response in said animal. Preferred doses are as hereinbefore described.

Where one antigen is employed desirably the antigen is substantially free from other membrane antigens.

Examples of particular parasitic nematodes include Trichinella, Ancylostoma, Strongylus, Trichostrongylus, Haemonchus, Ostertagia, Ascaris, Toxascaris, Uncinaria, Trichuris, Dirofilaria, Toxocara, Necator, Enterobius, Strongyloides and Wuchereria. Examples of such species include Trichinella spiralis, Ancylostoma caninum, Strongylus vulgaris, Trichostrongylus colubriformis, Haemonchus contortus, Ostertagia ostertagi, Ascaris suum, Toxascaris leonina, Uncinaria stenocephala, Trichuris vulpis, Dirofilaria immitis, Toxocara spp, Necator americanus, Ancylostoma duodenale, Ascaris lumbricoides, Trichuris trichiura, Enterobius vermicularus, Strongyloides stercoralis and Wuchereria bancrofti.

Preferably the parasitic nematode is *Haemonchus contortus.* Preferably the membrane associated antigens are native H-gal-GP and/or H11 antigens known in the art. For other parasitic nematodes, preferred antigens are species-specific homologues of H-gal-GP or H11, as easily identified by the skilled addresses through homology. Typically the homologues will display a high degree of homology.

Mutants of the aforementioned antigen may also be envisaged and may be used. Such mutants including one or more substitutions, inversions, deletions and/or additions in amino acid sequence, whilst still displaying similar functional activity. Again such mutants are likely to be highly homologous to the native protein to which they correspond. Thus, as well as the use of the native wild-type antigens, the present invention extends to the use of homologues and mutants which are at least 80%, such as at least 90%, 95% or 98% identical in sequence to the native wild-type antigen.

It is to be understood that the present invention relates to native antigens isolated and purified from the parasitic nematode and does not therefore extend to the use of recombinantly expressed antigens.

The immunogenic formulations of the present invention may further comprise an adjuvant. The term "adjuvant" as used herein refers to an agent used to enhance the immune response of the immunised host to the immunising composition.

The formulations of the present invention may be administered parenterally or potentially via mucosal routes in dosage unit formulations containing conventional, non-toxic, pharmaceutically and/or veterinarally acceptable carriers, diluents, adjuvants and/or excipients as desired.

Injectable formulations, for example, sterile injectable aqueous or oleaginous suspensions may be formulated according to the known art using suitable dispersing or wetting agents and suspending agents. The sterile injectable preparation may also be a sterile injectable solution or suspension in a non-toxic parenterally acceptable diluent or solvent, for example, a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suitable adjuvants for the vaccination of animals include but are not limited to oil emulsions such as Freund's complete or incomplete adjuvant (not suitable for livestock use), Marcotl 52: Montanide 888 (Marcol is a Trademark of Esso. Montanide is a Trademark of SEPPIC, Paris), squalane or squalene, Adjuvant 65 (containing peanut oil, mannide monooleate and aluminium monostearate), mineral gels such as aluminium hydroxide, aluminium phosphate, calcium phosphate and alum, surfactants such as hexadecylamine, octadecylamine, lysolecithin, dimethyldioctadecylammonium bromide, N,N-dioctadecyl-N', N'-bis(2-hydroxyethyl) propanediamine, methoxyhexadecylglycerol and pluronic polyols, polyanions such as pyran, dextran sulfate, polyacrylic acid and carbopol, peptides and amino acids such as muramyl dipeptide, dimethylglycine, tuftsin and trehalose dimycolate. The antigens, of the present invention can also be administered following incorporation into liposomes or other micro-carriers, or after conjugation to polysaccharides, proteins or polymers or in combination with Quil-A to form "Iscoms" (Immunostimulating complexes). Other adjuvants suitable for use in the present invention include conjugates comprising the immunogen together with an integral membrane protein of prokaryotic origin, such as TraT.

By "immunogenic", it is meant that there is a protective effect in the target animal which includes prevention of nematode infection, viability and/or fecundity as well as reduced infection, growth, viability and/or fecundity and also includes various levels of amelioration of symptoms of helminth infection.

Even a partial reduction in nematode numbers or ability to spread is useful in controlling nematode infection in animals. The protective effect can also be measured by increased productivity of a group of animals.

Preferred animals include sheep, goats, cattle and wild ruminants.

The administration of the immunogenic formulation may be for either a "prophylactic", or "therapeutic" purpose. When providing prophylactically, the immunogenic composition is provided in advance of any symptoms of an infection. The prophylactic administration of the immunogenic composition serves to prevent or attenuate any subsequent infection.

In order to protect an animal from *H. contortus* infection, an immunogenic composition of the present invention is administered to the animal in an effective manner such that the composition is capable of protecting that animal from infection. For example, it is able to elicit (i.e., stimulate) an immune response, preferably including both a humoral and cellular response, that is sufficient to protect the animal from infection.

A composition is said to be "pharmacologically acceptable" if its administration can be tolerated by a recipient animal and is otherwise suitable for administration to that animal. Such an agent is said to be administered in a "therapeutically effective amount" if the amount administered is physiologically significant. An agent is physiologically significant if its presence results in a detectable change in the physiology of a recipient animal.

The immunogenic formulations of the invention can be administered by either single or multiple dosages of an effective amount. Preferably two doses are administered. The formulations of the present invention may be administered alone or conjointly with other immunogenic formulations. By conjointly is meant that the different immunogenic formulations may be administered separately, but at substantially similar time points, or indeed the different formulations may be formulated together in a single multi-valent formulation. For example, the formulations of the present invention may be administered conjointly with a clostridial vaccine, such as, Convexin 8. Conjoint administration may have a synergistic effect on the efficacy of one or more of the immunogenic formulations.

Typically, a primary immunisation is given followed by one or more booster immunisation given 2 - 8 weeks apart. Injection, intramuscularly or sub-cutaneously are typical modes of administration. Other modes of administration are contemplated by the present invention and include intranasal, intraperitoneal, intrathecal, rectal, infusion and intrapulmonary administration. Administration may also be by nasal drip, aerosol, infusion through the skin or membrane surfaces or ingestion.

As would be understood by one of ordinary skill in the art, when the formulations of the present invention are provided to an animal, it may be in a composition which may contain salts, buffers, adjuvants, or other substances which are desirable for improving the efficacy of the composition.

Compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption. Liquid dosage forms for oral administration may generally comprise a liposome solution containing the liquid dosage form. Suitable forms for suspending liposomes include emulsions, suspensions, solutions, syrups, and elixirs containing inert diluents commonly used in the art, such as purified water. Besides the inert diluents, such compositions can also include adjuvants, wetting agents, emulsifying and suspending agents, or sweetening, flavouring, or perfuming agents.

The present invention will now be further described by way of example and with reference to the following Figures which show:
Figure 1 shows an SDS-PAGE profile of the H-gal-GP preparation;
Figure 2 is a graph showing group mean and standard error anti-H-gal-GP serum antibody concentrations as determined by ELISA of a first trial. The dose of H-gal-GP used to immunise each group on each vaccine day is shown. Arrows depict the vaccination times (labelled V1, V2 or V3 or the day of challenge (labelled Ch);
Figure 3 is a graph showing group mean and standard error faecal egg counts of the sheep following challenge with Haemonchus of the first trial. The dose of H-gal-GP used to immunise each group on each vaccine day is shown.
Figure 4 is a graph showing group mean and standard error anti-H-gal-GP serum antibody concentrations as determined by ELISA of second trial. The dose of H-gal-GP used to immunise each group on each vaccine day is shown. Arrows depict the vaccination times (labelled V1, V2 or V3) or the day of challenge (labelled Ch);

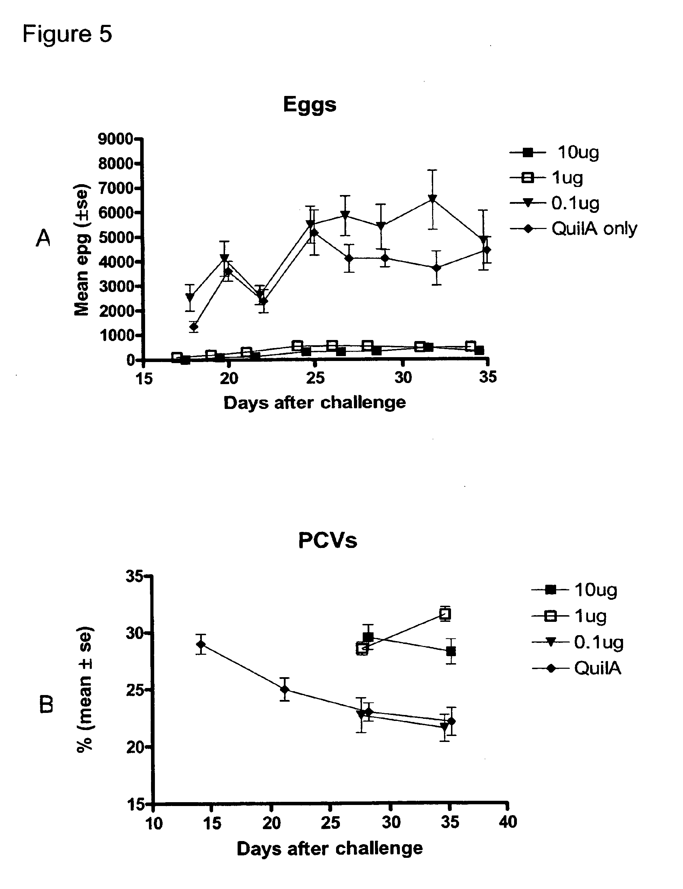

Figure 5A is a graph showing group mean and standard error faecal egg counts of the sheep following challenge with Haemonchus/ of the second trial;
Figure 5B is a graph showing group mean and standard error blood packed cell volumes of the sheep following challenge with Haemonchus of the second trial. The dose of H-gal-GP used to immunise each group on each vaccine day is shown on each graph.
Figure 6 shows a comparison of the SDS-PAGE profiles of H-gal-GP and H11 under non-reducing conditions. This was batch 120 of H11 used in Trial 3. Small amounts of H-gal-GP are observed in the H11 preparation (seen as shared bands at ~250 and ~42 kDa).
Figure 7 is a graph showing group mean and standard error faecal egg counts of the sheep following challenge with Haemonchus of the third trial. The dose of H-gal-GP used to immunise each group on each vaccine day is shown.
Figure 8 is a graph showing group mean and standard error faecal egg counts of the sheep following challenge with Haemonchus of the third trial. The dose and batch of H-gal-GP or H11 is identified.
Figure 9 is a graph showing group mean and standard error faecal egg counts of the sheep following challenge with Haemonchus of the third trial. The dose and batch of H11 is identified.
Figure 10 is a graph showing Anti-H-gal-GP antibody response of sheep immunised sub-cutaneously twice four weeks apart with 1µg of QuilA in trial 4.

### Materials and Methods

### Sheep and Design of Vaccine Trials

Six trials were carried out. Each contained sheep born and reared indoors at Moredun Institute, Edinburgh, UK under conditions designed to exclude nematode parasites and fed a maintenance ration of concentrates and hay.

### Trial 1

The purpose of this trial was to determine whether vaccines containing either 30 or 10 ug of native H-gal-GP per dose were protective.

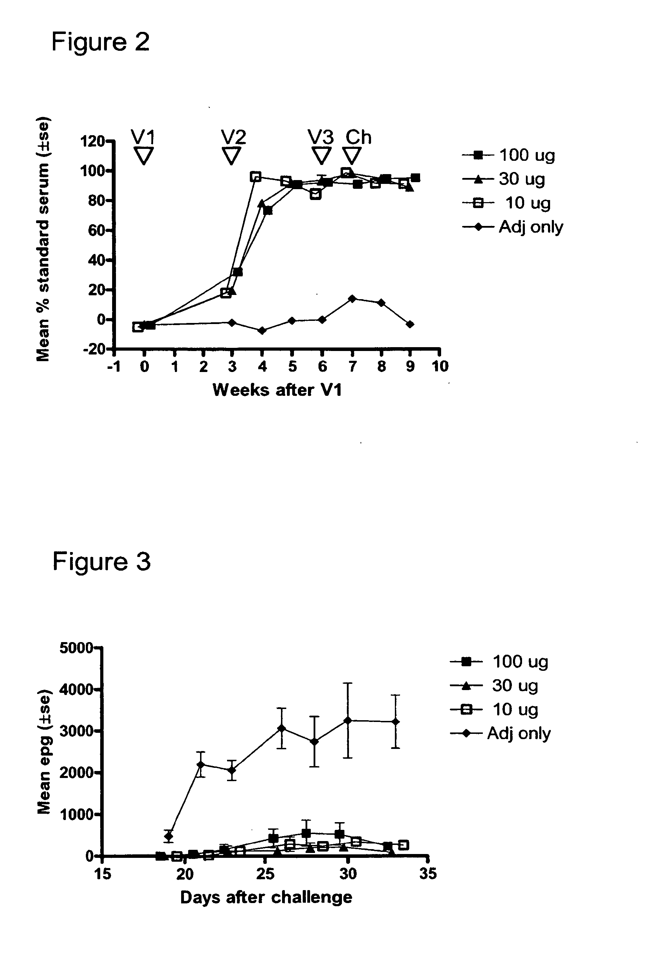

The trial contained 28 castrated Blackface x Leicester male lambs aged nine months, weighing between 35 and 52 Kg at the time of the first vaccination and allocated into 4 groups of 7 balanced for weight. The sheep were immunised three times, three weeks apart. On each vaccination day each sheep in each group was either given the dose of H-gal-GP shown in Figure 3 or given adjuvant alone. The H-gal-GP antigen (Batch 119) was diluted with a QuilA (Brenntag, Denmark) phosphate buffered saline solution so that each lamb received the appropriate dose together with 5mg QuilA on each vaccine day. The vaccine was administered intramuscularly as a 2ml dose, 1 ml being injected into each back leg.

### Trial 2

In view of the results of Trial 1, Trial 2 was conducted to determine whether the protective dose of native H-gal-GP could be as low as 1 or 0.1 ug.

The second trial used 28 Dorset x Suffolk lambs, aged 3 months and weighing between 27 and 46 Kg at the time of first vaccination. They were allocated to 4 groups of 7 balanced for weight and sex.

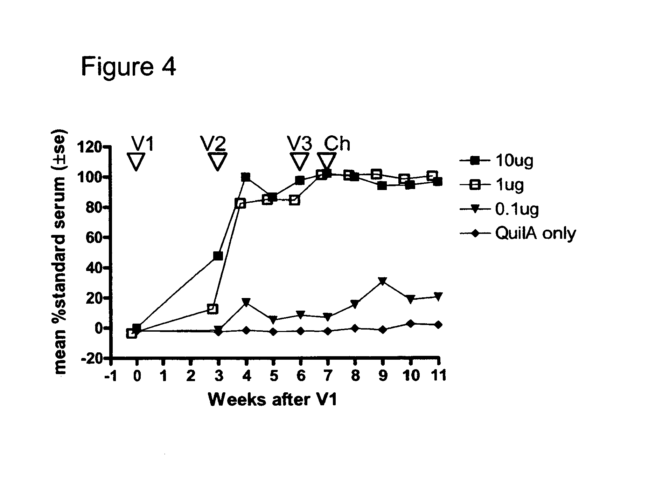

On each vaccination day each sheep in each group was either given the dose of H-gal-GP shown in Figure 4 or given adjuvant alone.The H-gal-GP antigen (Batch 119) was diluted with a QuilA (Brenntag, Denmark) phosphate buffered saline solution so that each lamb received the appropriate dose together with 5mg QuilA on each vaccine day. The vaccine was administered intramuscularly as a 2ml dose, 1 ml being injected into each back leg.

### Trial 3

There were three objectives with this trial:
1) to determine where within the 0.1 to 1 ug range the minimum protective dose of native H-gal-GP lay;
2) to determine whether a different batch of H-gal-GP was also protective when used at 1ug per dose; and
3) to determine whether H11 was also protective in the 1ug range.

Fifty six Suffolk x Blackface lambs aged 5 months and weighing between 29 and 44 Kg at the time of first vaccination were allocated to 8 groups of 7. Groups 1 to 4 were immunised with H-gal-GP, Groups 5 to 7 were injected with antigen enriched for H11 and Group 8 were the controls which received adjuvant alone. On each immunisation day Groups 1, 2 and 3 were given respectively 1, 0.6 or 0.3 ug of H-gal-GP from the same batch (119) used in Trial 2, whereas Group 4 were injected with 1ug of a different preparation (batch 120). On each immunisation day Groups 5, 6 and 7 were injected respectively with 10, 1 or 0.1 ug of the H11 enriched antigen made from the same extract as Group 4.

All antigens were diluted with a QuilA (Brenntag, Denmark) phosphate buffered saline solution so that each lamb received the appropriate dose together with 5mg QuilA on each vaccine day. The vaccine was administered intramuscularly as a 2ml dose, 1 ml being injected into each back leg.

### Trial 4

This trial was conducted mainly to determine whether the serological response of sheep immunised subcutaneously with 1ug H-gal-GP (batch 119) in 1mg QuilA was similar to those in Trials 2 and 3 which were given the same dose of the same batch of antigen but with 5mg of adjuvant intramuscularly. Further purposes of the trial were 1) to monitor any lesions which developed at the immunisation sites and 2) to determine the duration of the anti-H-gal-GP antibody response after two immunisations.

Seven Suffolk x Blackface lambs aged 6 months and weighing between 35 and 46 Kg at the time of first vaccination were immunised with 1 ug H-gal-GP (batch 119) in 1 mg QuilA. The innoculum volume was 1ml. It was injected subcutaneously behind one ear. These animals were not challenged with Haemonchus.

### Trial 5

This trial was done 1) to determine the minimum protective dose of ConA binding antigen (which is mainly a mixture of H-gal-GP and H11) in young lambs and 2) to determine whether simultaneous administration of a Clostridia vaccine affected either the immune response or the degree of protection conferred by a 1 ug dose. Six groups of 7 Greyface x Texel lambs aged 4 to 6 weeks at the time of first immunisation and weighing between 7 and 14 kg each were immunised with ConA binding antigen. Two of these groups were immunised with 1 ug of antigen per dose, the rest were given 5, 2, 0.5, or 0.25ug. All sheep received a 1ml subcutaneous innoculum behind the ear containing 1 mg QuilA and the ConA binding antigen 3 times 3 weeks apart. One of the 1 ug groups was also immunised with the clostridial vaccine "Covexin 8" at the same time as the first and third injection of ConA binding antigen. This was given as a 2ml dose behind the contralateral ear.

### Trial 6

This trial had three main objectives 1) to determine whether the ConA binding antigen was more protective if it was stored at 4C rather than being frozen at - 20C, 2) to confirm whether simultaneous administration of clostridium vaccine does enhance the immunogenicity of the Haemonchus antigen and, if so, 3) to determine the minimum dose of the ConA binding antigen when given simultaneously with the clostridial vaccine.

Six groups of 7 Greyface x Border Leicester lambs aged 4 to 6 weeks at the time of first immunisation and weighing between 7 and 14 kg each were immunised with ConA binding antigen. A further 3 groups of 7 acted as challenge controls. The controls (Groups 7, 8 and 9) and Groups 1, 2 and 3 were immunised 3 times 3 weeks apart, whereas Groups 4 , 5 and 6 were vaccinated twice (see Table 3). Groups 1 to 3 were all immunised with 1 ug ConA antigen per dose, which had either been stored frozen (Group 1) or at 4C (Groups 2 and 3). Groups 1 and 2 were immunised with Covexin 8 given at the time of their first and third injection only.

Groups 4, 5 and 6 formed a dose titration sub-trial and were given 5, 2 or 1 ug of ConA antigen, respectively.

All the control groups were injected with QuilA adjuvant but Group 7 was given Covexin as well. Group 9 was immunised with bacteriophage proteins which were not related to the Haemonchus ConA binding antigen. The route and dose of antigen were as determined in Trial 4.

In all experiments blood samples were obtained for serology on most weeks. All sheep were challenged with 5,000 Haemonchus larvae one week after the final immunisation, except in Trial 4 where the sheep were not challenged. Faecal egg counts were made 3 times per week from about day 18 after challenge. In Trial 2 heparinised blood for packed cell volumes was obtained at weekly intervals from two weeks after challenge in the control sheep and from 4 weeks post challenge in the immunised groups. In trials 1 and 2 the sheep were killed for worm counts 5 weeks post challenge.

### Parasitology procedures

Infective larvae were from a strain of H. *contortus,* which has been maintained at Moredun for several years.

The methods for faecal egg counting, enumeration of worm burdens and methods for obtaining clean adult parasites for protein preparations have been described before (Smith and Smith 1993).

### Preparation of H-gal-GP

This was the peanut binding fraction of a detergent extract of adult H. *contortus* membranes, prepared exactly as described before (Smith et al 2001). The same preparation (batch 119) was used in Trials 1 to 4 except in one Group in Trial 3 where the subsequent batch (120) was used. H-gal-GP was stored at - 20C in the intervals between these four trials.

### Preparation of H11 enriched fraction

A detergent extract of adult *H. contortus* membranes, prepared exactly as described before (Smith et al 2001), was pumped through 3 columns connected in series containing agarose beads coupled with either peanut, jacalin or ConA lectins in that sequence. After thorough washing with buffer, the columns were disconnected and eluted separately with an appropriate sugar solution. The fraction eluted from the ConA column with 0.5M methylmannoside was the H11 enriched preparation.

### Preparation of ConA binding antigen

This was done as just described for the H11 preparation except that the peanut and jacalin columns were omitted from the process. Therefore the fraction eluted from the ConA column also contained proteins which would have bound to peanut (H-gal-GP) and jacalin lectins (H-sialgal-GP) (Smith et al 2000).

### Protein estimation of the H-gal-GP preparation

Bovine serum albumin (BSA) standards were prepared by dilution of the 2 mg/ml sample provided in the PIERCE BCA Protein Assay Kit 23225 with phosphate buffered saline (PBS) to 1:2, 1:3 and 1:4 and stored at 4°C. The H-gal-GP preparation was diluted in the same way and 10µl of each standard and each H-ga-IGP dilution was added in triplicate to wells of a microtitre plate. Immediately, 200µl freshly prepared bicinchoninic acid (BCA Working Reagent) was added to each well using a multichannel pipette and the plate was covered. Following incubation at 37°C for 30 minutes, the absorbance at 562 nm was measured. When the absorbance readings for the BSA standards were plotted against their respective concentrations a linear relationship was obtained and the best fit calculated to form a standard curve. The dilution of H-gal-GP which gave an OD value close to the average standard was chosen and its concentration determined from the standard curve. Eight repeats of this assay were conducted with and the mean and standard deviation obtained was 3863.91 ± 353.9µg/ml.

The same technique was used to measure the protein content of the H11 and ConA binding preparations.

### Measuring serum to antibodies to Haemonchus vaccine antigens by ELISA

Antibodies specific for the immunogen used in each experiment were measured. Thus, for example, anti-H-gal-GP antibodies were assayed in Trials 1 and 2 and anti-ConA binding antigen antibodies were assayed in Trials 5 and 6. In Trials 1 and 2 anti-H-gal-GP antibodies were measured by an ELISA which has been described before (Smith et al 2000). All samples were expressed as a percent of a standard immune serum sample.

A titration end point ELISA was used for the sera obtained in the other trials. ELISA plates were coated by filling each well with 50ul of antigen diluted to 1 ug / ml in 50mM Sodium bicarbonate buffer, pH 9.6 and incubated overnight at 4C.

Sera were diluted to 1/100 in TNTT wash buffer (10mM Tris/HCl, pH7.4 containing 0.5M NaCl, 0.05% v/v Tween 20) and doubling dilutions were then prepared to 1/204,800, using separate low protein binding micro titre plates. Negative controls for each plate were prepared from a pool of pre-immunisation sera taken from the group of animals under test and diluted 1/200 in wash buffer. A standard plate wash consisted of 6 cycles using a plate washing machine.

Antigen coated plates were washed once then blocked by filling with 10% Infasoy powdered milk in wash buffer and incubating overnight at 4C. The plates were washed once, then 50ul of each dilution of the test and control sera were added per well and incubated for 1 hr at room temperature. After another wash, the wells were filled with 50ul mouse monoclonal anti sheep IgG-HRPO conjugate (Sigma cat# A9452) diluted 1/10,000 in TNTT and incubated for 1 hr at room temperature. After a further wash, 50ul of OPD substrate (Sigma cat# P9187) was added to each well and incubated in the dark for 20 min at room temperature. The colour reaction was stopped by the addition of 25ul of 2.5 M sulphuric acid to each well. The plates were then read at 490nm using an appropriate ELISA plate reader. The titres of the samples were expressed as that dilution of the serum required to give the same OD ₄₉₀ as the negative control sample diluted 1/200. Titres were calculated by non-linear regression using Microsoft Excel to fit a second order polynomial curve to a double log plot of dilution factor against A₄₉₀.

### SDS PAGE analysis of Haemonchus antigens

Briefly, 10ug of antigen was separated on a 5-15% gradient acrylamide gel using a modification of the procedure of Laemmli (1970). The protein marker lane was loaded with 5µl of a protein ladder (Fermentas PageRuler sm0661). Both samples were heated at 100°C for 3 min in an equal volume of 63mM Tris-HCl pH 6.8 containing 5% w/v SDS without a reducing agent. The gel was run at 200V for 60 minutes then stained with Coomassie Blue.

### Packed cell volumes (PCVs)

Heparinised blood was aspirated into small glass capillary tubes which were then sealed at one end by melting the glass in the flame of a Bunsen burner. The tubes were spun in a microhaematocrit centrifuge for 15 mins and the PCVs determined with a reader designed for this task (Hawksley)

### Results

### H-gal-GP antigen preparation used in low-dose Trials 1 and 2

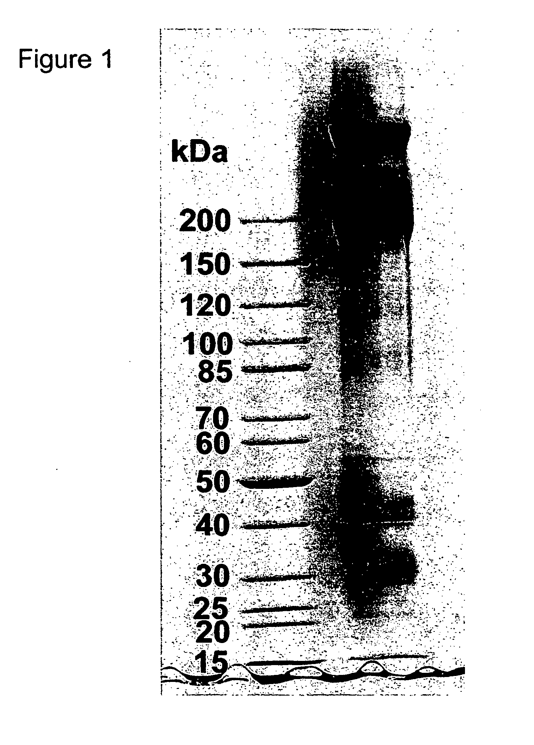

The protein profile of this antigen (batch 119) closely resembled that of previously published preparations (Fig 1) (Smith at al 1999).

### Trial 1.

### 1) Introduction

The purpose of this trial was to determine whether vaccines containing either 30 or 10 ug of native H-gal-GP per dose were protective.

### 2) Results

### a) serology

Group mean anti H-gal-GP antibody responses are illustrated in Fig 2. OD values close to 0 were always detected in the adjuvant only control group except on Weeks 7 and 8 when values reached about 0.1.

At the start of the trial the ODs of the H-gal-GP immunised sheep were indistinguishable from the adjuvant only group. By the time of the second immunisation, all 3 immunised groups showed a rise in OD, which increased sharply by Week 4, 7 days after the first vaccine boost. The mean OD values of all three antigen groups remained above 0.9 from Week 5 to the end of the trial, with no discernable difference between these groups.

### b) parasitology

Group mean faecal egg counts are plotted in Fig 3. From Day 21, worm egg counts were substantially reduced in all 3 immunised groups compared to the controls which received adjuvant only. Statistically, there was no difference between the cumulative total egg counts of the different antigen dose groups, with mean overall reductions in egg counts ranging from 88.5 to 95.1%.

Group mean worm counts (with Standard Errors) were 779 (199), 468 (163), 843 (256) and 2813 (238) for the 100, 30, 10 and adjuvant only groups, respectively. Thus all three vaccinated groups had markedly fewer parasites compared to the controls which received adjuvant only.

### Trial 2

### 1) Introduction

The purpose of this trial was to determine whether vaccines containing either 10, 1 or 0.1 ug of native H-gal-GP per dose were protective.

### 2) Results

### a) serology

Group mean anti H-gal-GP antibody responses are illustrated in Fig 4. OD values close to 0 were always detected in the adjuvant only control group.

At the start of the trial the ODs of the H-gal-GP immunised sheep were indistinguishable from the adjuvant only group. By the time of the second immunisation, only the 10 and 1ug groups showed a rise in OD, which was more pronounced in the 10ug group. However, by Week 4, 7 days after the first vaccine boost, the mean OD values for the 10 and 1 ug groups rose sharply to values similar to those recorded in the Trial 1. The antibody concentrations in both these vaccinated groups were very similar at this time and remained so for the duration of the trial. Meanwhile the values of the 0.1 ug group were substantially lower, though clearly elevated compared to the controls.

### b) parasitology and PCV data

Over the course of the trial the 10 and 1µg dose groups shed ~90% fewer Haemonchus eggs than either the 0.1µg or the adjuvant only groups (Fig 4a). There were no obvious differences in egg output between the 10 and 1µg doses, nor between the 0.1 ug and control groups.

Group mean worm counts (with Standard Errors) were 415 (198), 816 (2224), 2255 (506) and 1563 (380) for the 10, 1, 0.1 and adjuvant only groups, respectively. Thus the 10 and 1 ug vaccinated groups had fewer parasites than the 0.1 ug group or the controls which had received adjuvant only.

The PCVs of the control sheep gradually declined from a mean of 29.0% two weeks post challenge to 22.1% on week 4 (Fig 4B). The PCVs of the 01µg group were very similar to those of the controls 4 and 5 weeks post challenge indicating that both were anaemic. In contrast mean values in the 10 or 1µg vaccinated sheep were significantly higher at these times and remained witf normal ovine range (28-40%).

### Conclusions from Trials 1 and 2.

The results of the foregoing trials clearly demonstrated that H-gal-GP doses of between 100 to 1µg could reduce the egg output from a Haemonchus challenge by some 90%. In Trial 2 the 10 and 1ug doses also prevented the anaemia which developed in the control sheep from three weeks post challenge.

Three immunisations were used in these experiments but the serology results in both trials clearly showed that the antibody responses did not increase significantly after the third injection, suggesting that two injections would have sufficed. In a previous study sheep immunised twice or three times with 100ug H-gal-GP / injection were equally well protected (unpublished data).

If a dose of, for example, 1µg is used to vaccinate ruminants, such as sheep, it becomes commercially viable to prepare a native vaccine. For example, by deliberately infecting suitable lambs with an appropriate dose of Haemonchus larvae, it is possible to harvest 10g of adult worms from each sheep 3 weeks later. Approximately 0.5mg of H-gal-GP can be purified from 1g of adult Haemonchus. Therefore a single donor sheep can provide 5 mg H-gal-GP, the equivalent of 5,000 doses of vaccine.

### Trial 3

### 1) Introduction

This trial was conducted, first, to determine where within the 0.1 to 1 ug range the minimum protective dose of native H-gal-GP lay; second, to determine whether a different batch of H-gal-GP was also protective when used at 1 ug per dose and, third, to determine whether H11 was also protective in the low ug range.

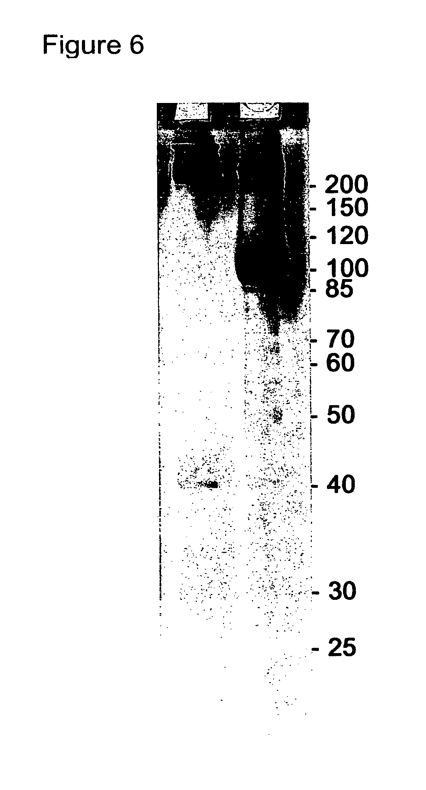

While native H-gal-GP can readily be prepared free of H11, it is much more difficult to make native H11 free of H-gal-GP (Fig 6). This and the finding that a cocktail of recombinant H11 aminopeptidases are not protective, despite being enzymically active, have called into question whether H11 is a genuinely protective antigen per se (Smith and Zarlenga, 2006). Could the protection conferred by native H11 preparations actually have been caused by contaminating H-gal-GP? When H11 is prepared as specified above, it is estimated that H-gal-GP represents about 10% of the total protein (Fig 6). Therefore, if a 1 ug dose of H11 was protective, the effect could not be ascribed to H-gal-GP, because it was shown in Trial 2 that 0.1 ug H-gal-GP does not protect.

### 2) Results

### a) faecal egg counts

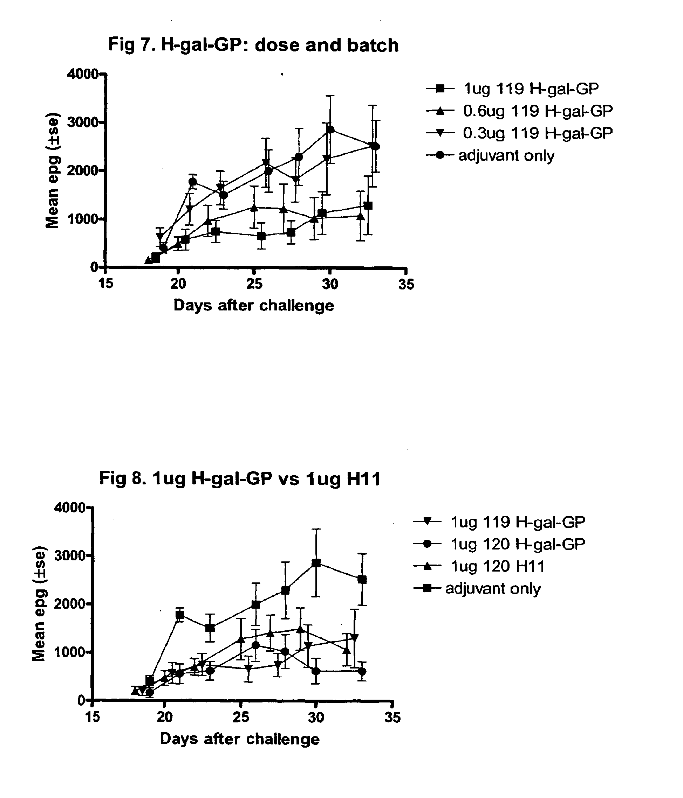

Over the course of the trial the groups immunised with 1 and 0.6ug of Batch 119 H-gal-GP shed some 59 or 54% fewer worm eggs respectively than the adjuvant only control group (Fig 7). The group immunised with 0.3ug was not protected to any useful extent (5% reduction in egg count). The group immunised with 1 ug of Batch 120 H-gal-GP shed 63.8% fewer eggs suggesting it and Batch 119 were equally protective (Fig 8).

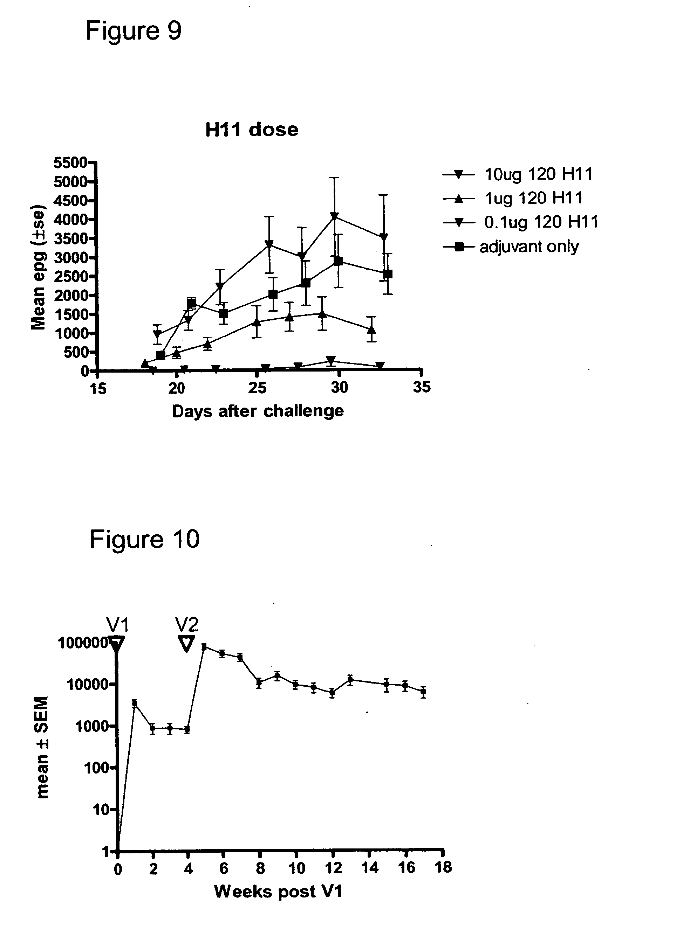

Immunisation with 10ug of the H11 preparation severely depressed worm egg output (Fig 9), so that this group shed 96.4% fewer eggs than the controls over the duration of the trial. One ug of the same preparation was less effective (48.9% reduction) and the 0.1 ug dose did not protect at all (Fig 9).

One ug doses of either H-11 or H-gal-GP were approximately equally protective (48.9 vs 58.6% epg reduction, respectively) as shown in Fig 8.

### 3) Conclusions from Trial 3.

For unknown reasons the protective effects observed with H-gal-GP were not as strong as those detected in Trials 1 and 2. Nevertheless a 0.6ug dose of H-gal-GP still depressed egg output substantially suggesting that the minimum protective dose lay somewhere between 0.3 and 0.6ug per injection.

A different batch of H-gal-GP was just as effective at 1ug, suggesting that manufacturing this protective antigen could be a reliable process. The finding that 1 ug doses of both H11 and H-gal-GP were approximately equally protective, strongly suggested that native H11 is a genuinely protective antigen and that the effect of such a preparation could not just be attributed to contaminating H-gal-GP. This result also raised the possibility that these two antigens might act additively or synergistically and that it would be worth evaluating preparations containing a combination of them.

### Trial 4.

### 1) Introduction

The overall purpose of this trial was to progress the effects observed in the previous trials towards a practical vaccine for farmers. The dose of QuilA adjuvant was therefore reduced from 5 to 1 mg per vaccine dose and the 1 ug H-gal_GP vaccine was administered on two occasions four weeks apart, each time as a 1ml subcutaneous injection behind an ear. The animals were examined for a few days after vaccine administration to monitor any lesions which might have developed at the immunisation sites. The sheep in this trial were not challenged, rather their serological response was monitored for 13 weeks to determine the likely duration of immunity following this practical immunisation regime.

### 2) Results

### a) lesion monitoring

Transient mild swellings and or skin thickening were detected at the injection sites of some sheep for 3 or 4 days after vaccination.

### b) serology

The time course of the serum antibody titres of these sheep is shown in Fig 10. Antibody concentrations rose sharply after the booster immunisation to peak one week later. They then declined until about week 8, thereafter remaining well above pre-booster values for the rest of the trial.

Comparison of Trial 4 peak titres with those attained a week after the booster immunisation in trials 2 or 3, showed that using 1 mg of QuilA and giving the innoculum subcutaneously appeared to enhance the antibody response (Table 1)

**Table 1. Mean and standard error antibody titres one week after the second vaccination with 1 ug H-gal-GP in trials 2, 3 and 4.**

| Trial no (batch no) | Mean | Standard Error | Injection Route | Amount of QuilA (mg) |
|---|---|---|---|---|
| 2(119) | 38,461 | 19,735 | I/M | 5 |
| 3(119) | 19,133 | 6,695 | I/M | 5 |
| 3(120) | 14,053 | 2,348 | I/M | 5 |
| 4(119) | 78,619 | 13,362 | S/C | 1 |

### 2) Conclusions

The skin reactions resulting from immunisation were perfectly acceptable, being milder than those stimulated by other commercially available vaccines used in sheep.

Reducing the dose of QuilA from 5 to 1 mg and giving the vaccine subcutaneously rather than intramuscularly did not adversely affect the antibody response, rather it appeared to enhance it.

Using this immunisation regime, antibody titres persisted at well above pre-boost concentrations for about 14 weeks.

### Trial 5.

### 1) Introduction

In view of the results of Trial 3 which showed that both H-gal-GP and H11 were protective when used at 1ug doses, it was decided to test both antigens in combination in the hope that additive or synergistic effects might be observed. ConA binding antigen contains a combination of H-gal-GP and H11 and has the additional advantage of being simpler and cheaper to prepare than either H-gal-GP or H11 separately.

A practical vaccine for Haemonchus must be effective in weaned lambs, the class of sheep most susceptible to gastro-intestinal nematode infections. It would be ideal if the first dose of vaccine could be administered at "marking", when the lambs are gathered for tail docking and castration at about 4 to 6 weeks of age, and the second dose given at weaning time approximately 6 weeks later. However, a more conservative approach was adopted for Trial 5 so that a third immunisation was given between these two times. The amount of adjuvant, route and site of vaccination followed that determined in Trial 4.

Australian sheep farmers routinely vaccinate their lambs against Clostridial diseases at marking and weaning times. Clostridial vaccines are usually multivalent (ie they are effective against several strains of Clostridial bacteria) and contain a relatively crude mixture of bacterial toxoids and bacterins. If a Haemonchus vaccine was to be given at the same time as these, it was important to determine whether there would be any adverse interference in the subsequent immune response to the Haemonchus antigen.

### 2) Results

### a)Serology

Peak group mean serum anti-conA binding antigen titres are shown in Table 2. Titres in the adjuvant only control group were negligible throughout the trial. In contrast, significant titres were first detected after the second vaccination in all groups receiving antigen and these increased substantially after the third immunisation. However, there was little difference between the titres of the antigen groups, with the exception of the group which received Covexin 8, where the response was better.

**Table 2: Peak antibody titres, average worm egg outputs and percent protection in Trial 5**

| Group | Antigen | Titre | | epg | | % Protection | |
|---|---|---|---|---|---|---|---|
| | | mean | se | mean | se | mean | se |
| 1 | 5 ug | 5,538 | 1,067 | 1080.6 | 181.64 | 41.9 | 9.76 |
| 2 | 2 ug | 8,088 | 943 | 1278.7 | 257.70 | 31.3 | 13.85 |
| 3 | 1 ug | 6,439 | 734 | 1231.1 | 300.27 | 33.8 | 16.14 |
| 4 | 0.5 ug | 5,482 | 1,157 | 2299.1 | 526.70 | -23.6 | 28.31 |
| 5 | 0.25 ug | 5,151 | 1,086 | 1676.5 | 667.12 | 9.9 | 35.85 |
| 6 | 1 ug + covexin | 12,503 | 3,452 | 467.0 | 210.75 | 74.9 | 11.33 |
| 7 | adjuvant | 689 | 687 | 1860.6 | 247.43 | 0.0 | 13.30 |

### a) Faecal egg counts

Group mean egg counts averaged over 7 samplings taken between days 18 and 32 post challenge are shown in Table 2 together with their corresponding percent protection values (ie the relative reduction of the vaccinated group egg counts compared to the adjuvant only controls). The degree of protection conferred to the groups which were vaccinated with either 5, 2 or 1ug of antigen was similar ranging between 31.3 and 41.9% but those which were given 0.5 or 0.1 ug of antigen were not significantly protected. Mean % protection values for the group immunised with Covexin and 1 ug antigen were more than twice those of Group 3 which received the same dose of antigen alone.

### 3) Conclusions from Trial 5.

Three immunisations with 5, 2 or 1 ug of ConA binding antigen in QuilA adjuvant conferred significant (31 to 42%) reductions in worm egg output on young lambs. However, if Covexin 8 was injected at the same time as 1 ug of the same antigen, higher antibody titres were stimulated and protection increased to a mean of almost 75%.

The degree of protection conferred with 1ug ConA binding antigen alone on the young lambs in this experiment was lower than that detected with 1 ug of H-gal-GP or H11 with the older lambs used in Trial 3. It was not clear whether this difference was due to the age of sheep or to the difference in antigen used.

In connection with the second of these explanations, the ConA antigen preparation used in this trial had been frozen at -20C for a few weeks before it had been used. After the trial had been completed parallel studies with the ConA antigen showed that its H11 aminopeptidase activity was adversely affected by freezing. In contrast if the same preparation was stored at 4C, the aminopeptidase activity remained relatively constant.

### Trial 6

### 1) Introduction

This trial had three main objectives 1) to determine whether the ConA binding antigen was more protective if it was stored at 4C rather than being frozen at -20C, 2) to confirm whether simultaneous administration of clostridium vaccine does enhance the immunogenicity of the Haemonchus antigen and, if so, 3) to determine the minimum dose of the ConA binding antigen when given simultaneously with the clostridial vaccine.

### 2) Results

Four lambs died during the course of the trial from causes not related to the trial. Serum antibody titres at the time of challenge, mean faecal egg counts and percent protection values are all detailed in table 3.

### a) Serology

At the time the challenge infection was given all except four vaccinated lambs had titres in the thousands. No antibodies were detected in these individuals, all of which had been vaccinated only twice. Apart from a few with low titres, the control sheep in groups 7, 8 and 9 were also sero-negative. Three sheep which had received Covexin and antigen were the only ones with titres greater than 10,000.

### b) Egg counts

Individual and group mean egg counts averaged over the trial are depicted in Table 3. Percent protection values were calculated relative to an average of all three control groups. Storing the antigen at 4C seemed to be just as effective as freezing it at -20C. When three injections were given (Groups 1 to 3) the %protection values were similar whether Covexin was co-administered or not. Two injections of 1 ug with Covexin were as protective as three (Group 6 vs Group 3). However, if the non-responding sheep were excluded from the calculations, the data from groups 4, 5 and 6 indicated that two injections containing either 5 or 2ug antigen both with Covexin could reduce egg output by some 80%.

### c) Worm burdens

After the sheep were killed, their abomasa were pooled group by group and the adult Haemonchus were collected almost free from the stomach contents. The parasites were placed into graduated 50ml tubes so that the volume of worms collected could be measured. The abomasa of the four non-responder sheep identified in Table 3 as being sero-negative were not added to their group pools, rather they were processed individually. Thus the total volume of worms recovered from each group (less any non-responders) was obtained and the mean volume of worms recovered per lamb calculated, as displayed in Table 4. The results showed that the groups immunised twice with either 5 or 2 ug of antigen together with Covexin were best protected with burdens some 70% lower than control values. Much higher burdens, similar to those present in the controls were recovered from the four non-responder sheep.

### 3) Conclusions from Trial 6.

That substantial protection can be stimulated in young lambs vaccinated twice with either 5 or 2 ug of ConA binding antigen given at the same time that Covexin 8 is administered. However, a small proportion of lambs immunised in this way failed to respond for unknown reasons.

The antigen can be stored at 4C without any apparent deterioration compared with antigen stored frozen at -20C.

**Table 3. Trial 6: antibody titres and egg counts**

| **Group** | **Treatments** | | | | **Sheep** | **Antibody titre at challenge** | **Mean epg over trial** | **%Protection** | **%Protection without non responders** |
|---|---|---|---|---|---|---|---|---|---|
| | **Storage** | **Dose of ConA Ag** | **Covexin** | **no of Vs** | | | | | |
| | | | | | **1** | 8882 | 574 | 73.8 | 73.8 |
| | | | | | **2** | 6953 | 383 | 82.5 | 82.5 |
| | | | | | **3** | 9452 | 300 | 86.3 | 86.3 |
| 1 | -20 | 1 | + | 3 | **5** | 5584 | 2082 | 5.2 | 5.2 |
| | | | | | **6** | 3542 | 833 | 62.1 | 62.1 |
| | | | | | **7** | 7778 | 2967 | -35.2 | -35.2 |
| | | | | | **mean** | | **1189.8** | **45.8** | **45.8** |
| | | | | | | | | | |
| | | | | | **8** | 3250 | 2075 | 5.4 | 5.4 |
| | | | | | **10** | 4475 | 40 | 98.2 | 98.2 |
| | | | | | **11** | 3331 | 1019 | 53.6 | 53.6 |
| 2 | 4 | 1 | + | 3 | **12** | 4135 | 290 | 86.8 | 86.8 |
| | | | | | **13** | 1914 | 1682 | 23.4 | 23.4 |
| | | | | | **14** | 3275 | 2194 | 0.1 | 0.1 |
| | | | | | **mean** | | **1216.7** | **44.6** | **44.6** |
| | | | | | **15** | 4790 | 1058 | 51.8 | 51.8 |
| | | | | | **17** | 3711 | 880 | 59.9 | 59.9 |
| 3 | 4 | 1 | - | 3 | **19** | 6670 | 794 | 63.8 | 63.8 |
| | | | | | **20** | 1341 | 2352 | -7.2 | -7.2 |
| | | | | | **21** | 4881 | 1157 | 47.3 | 47.3 |
| | | | | | **mean** | | **1248.28** | **43.1** | **43.1** |
| | | | | | | | | | |
| | | | | | **22** | 7079 | 337 | 84.7 | 84.7 |
| | | | | | **23** | 5822 | 44 | 98.0 | 98.0 |
| | | | | | **24** | 0 | 4130 | -88.1 | |
| 4 | 4 | 5 | + | 2 | **25** | 5591 | 1118 | 49.1 | 49.1 |
| | | | | | **26** | 12581 | 27 | 98.8 | 98.8 |
| | | | | | **27** | 4745 | 212 | 90.3 | 90.3 |
| | | | | | **28** | 5912 | 590 | 73.1 | 73.1 |
| | | | | | **mean** | | **923** | **58.0** | **82.3** |
| | | | | | | | | | |
| | | | | | **29** | 6819 | 231 | 89.5 | 89.5 |
| | | | | | **30** | 5036 | 85 | 96.1 | 96.1 |
| | | | | | **31** | 1775 | 809 | 63.2 | 63.2 |
| 5 | 4 | 2 | + | 2 | **32** | 0 | 2352 | -7.2 | |
| | | | | | **33** | 0 | 1141 | 48.0 | |
| | | | | | **34** | 3162 | 461 | 79.0 | 79.0 |
| | | | | | **35** | 13142 | 735 | 66.5 | 66.5 |
| | | | | | **mean** | | **831** | **62.2** | **78.9** |
| | | | | | **36** | 7122 | 773 | 64.8 | 64.8 |
| | | | | | **37** | 3274 | 1045 | 52.4 | 52.4 |
| 6 | 4 | 1 | + | 2 | **38** | 0 | 3103 | -41.3 | |
| | | | | | **39** | 23800 | 416 | 81.1 | 81.1 |
| | | | | | **40** | 3913 | 1943 | 11.5 | 11.5 |
| | | | | | **41** | 2885 | 375 | 82.9 | 82.9 |
| | | | | | **42** | 4773 | 1920 | 12.5 | 12.5 |
| | | | | | **mean** | | **1368** | **37.7** | **50.9** |
| | | | | | | | | | |
| | | | | | **43** | 0 | 2354 | | |
| | | | | | **44** | 0 | 1799 | | |
| | | | | | **46** | 0 | 1069 | | |
| 7 | | 0 | + | 3 | **47** | 0 | 1112 | | |
| | | | | | **48** | 0 | 1697 | | |
| | | | | | **49** | 59 | 709 | | |
| | | | | | **mean** | | **1457** | | |
| | | | | | | | | | |
| | | | | | **50** | 0 | 723 | | |
| | | | | | **51** | 0 | 2641 | | |
| 8 | | 0 | - | 3 | **52** | 0 | 2744 | | |
| | | | | | **53** | 0 | 1601 | | |
| | | | | | **54** | 0 | 1649 | | |
| | | | | | **55** | 12 | 5482 | | |
| | | | | | **56** | 124 | 1323 | | |
| | | | | | **mean** | | **2309** | | |
| | | | | | 57 | 0 | 3005 | | |
| | | | | | 58 | 56 | 2891 | | |
| 9 | | 0 | - | 3 | 59 | 39 | 4619 | | |
| | | | | | 60 | 0 | 1930 | | |
| | | | | | 61 | 0 | 1112 | | |
| | | | | | 62 | 0 | 3027 | | |
| | | | | | 63 | 0 | 3151 | | |
| | | | | | **mean** | | **2819** | | |
| | | | | | | | | | |
| **Mean epg of groups 7,8 and 9 for percent protection calculation** | | | | | | | | | 2195.0 |

**Table 4. Trial 6 Worm Burdens**

| **Group no** | **Number of non responders** | **Volume of worms (ml)** | **Number of responders** | **Average vol of worms per responder** | **Mean vol of control groups** | **% Protection** |
|---|---|---|---|---|---|---|
| 1 | | 32 | 6 | 5.3 | | 45.0 |
| 2 | | 26 | 6 | 4.3 | | 55.3 |
| 3 | | 45 | 5 | 9.0 | | 7.2 |
| 4 | 1 | 16 | 6 | 2.7 | | 72.5 |
| 5 | 2 | 13 | 5 | 2.6 | | 73.2 |
| 6 | 1 | 25 | 6 | 4.2 | | 57.0 |
| 7 | | 57.5 | 7 | 8.2 | 9.7 | |
| 8 | | 112 | 7 | 16.0 | | |
| 9 | | 35 | 7 | 5.0 | | |
| | | | | | | |

| **Group no** | **Non responder sheep** | **Volume of worms ml** | | | | |
|---|---|---|---|---|---|---|
| 4 | 24 | 17 | | | | |
| | | | | | | |
| 5 | 32 | 10 | | | | |
| 5 | 33 | 10 | | | | |
| 6 | 38 | 8 | | | | |

### References

Barnes, E.H., Dobson, R.J., and Barger, I.A. Worm control and Anthelmintic Resistance: Adventures with a Model. Parasitology Today 1995; 11: 56-63.
Franzetti B, Schoehn G, Hernandez JF, Jaquinod M, Ruigrok RW, Zaccai G. Tetrahedral aminopeptidase: a novel large protease complex from archaea. EMBO J 2002 May 1;21(9):2132-8.
Jasmer DP, Perryman LE, Conder GA, Crow S, McGuire T. Protective immunity to Haemonchus contortus induced by immunoaffinity isolated antigens that share a phylogenetically conserved carbohydrate gut surface epitope. J Immunol 1993 Nov 15;151(10):5450-60.
Knox DP, Smith WD (2001). Vaccination against gastrointestinal nematode parasites of ruminants using gut-expressed antigens. Eighth European Multicolloquium of Parasitology. Veterinary Parasitology 100 21-32.
Knox DP, Redmond DL, Newlands GF, Skuce PJ, Pettit D, Smith WD (2003). The nature and prospects for gut membrane proteins as vaccine candidates for Haemonchus contortus and other ruminant trichostrongyloids. International Journal for Parasitology 33 1129-1137.
LeJambre LF, Windon RG, Smith WD (2008). Vaccination against Haemonchus contortus: Performance of native parasite gut membrane glycoproteins in Merino lambs grazing contaminated pasture. Veterinary Parasitology 153 (3-4) 302-312.
Muench S, Chun FS, Parcej D , Taylor S,Halliday AM, Newlands G, Smith WD and Trinick J. 3D Reconstruction of a Large Protease Complex from Haemonchus contortus Poster presented at US Biophysical Society conference, San Diego February 2008.
Newlands GF, Skuce PJ, Nisbet AJ, Redmond, D.L., Smith, S.K., Pettit, D., Smith, W.D. Molecular characterization of a family of metalloendopeptidases from the intestinal brush border of Haemonchus contortus. Parasitology 2006 Sep;133(Pt 3):357-68.
Newton SE, Meeusen EN. Progress and new technologies for developing vaccines against gastrointestinal nematode parasites of sheep. Parasite Immunol 2003 May;25(5):283-96.
Smith SK, Pettit D, Newlands GFJ, Redmond DL, Skuce PJ, Knox DP, Smith WD (1999). Further immunization and biochemical studies with a protective antigen complex from the microvillar membrane of the intestine of Haemonchus contortus. Parasite Immunology 21 187-199.
Smith, T.S., Munn, E.A., Graham, M., Tavernor, A.S., and Greenwood C.A. Purification and evaluation of the integral membrane protein H11 as a protective antigen against Haemonchus contortus. Int J Parasitol 1993; 23: 271-280.
Smith, WD & Smith, SK. Evaluation of aspects of the protection afforded to sheep immunized with a gut membrane-protein of Haemonchus contortus. Res Vet Sci, 1993; 55: 1-9.
Smith WD, Smith SK, Pettit D, Newlands GFJ, Skuce PJ (2000). Relative protective properties of three membrane glycoprotein fractions from Haemonchus contortus. Parasite Immunology 22 63-71.
Smith WD, Skuce PJ, Newlands GF, Smith SK, Pettit D. a) Aspartyl proteases from the intestinal brush border of Haemonchus contortus as protective antigens for sheep. Parasite Immunol 2003 Nov;25(11-12):521-30.
Smith WD, Newlands GF, Smith SK, Pettit D, Skuce PJ. b) Metalloendopeptidases from the intestinal brush border of Haemonchus contortus as protective antigens for sheep. Parasite Immunol 2003 Jun;25(6):313-23.
Smith WD, Zarlenga DS. Developments and hurdles in generating vaccines for controlling helminth parasites of grazing ruminants. Vet Parasitol 2006 Jul 31;139(4):347-59.)
Tamura T, Tamura N, Cejka Z, Hegerl R, Lottspeich F, Baumeister W. Tricorn protease--the core of a modular proteolytic system. Science 1996 Nov 22;274(5291):1385-9.

## Claims

1. An immunogenic formulation for use in a method for protecting and/or treating an animal against a parasitic nematode, wherein the immunogenic formulation comprises a purified integral intestinal cell membrane glycoprotein antigen from a nematode obtained from an infected animal wherein the intestinal cell membrane associated antigen is H-gal-GP and/or H11 antigen, or species specific mutant displaying similar functional activity thereof in an amount between 1µg to 10µg.

2. A process for preparing an immunogenic formulation for use in a method for protecting and/or treating an animal against a parasitic nematode, which process comprises:
b) isolating nematodes from an infected animal;
b) obtaining intestinal cell membranous material from said nematodes;
c) purifying an intestinal cell membrane associated antigen from said membranous material wherein the intestinal cell membrane associated antigen is H-gal-GP and/or H11 antigen, or species specific variant, mutant or homologue thereof, and
preparing said formulation comprising between 1µg to 10µg of said purified antigen.

3. An immunogenic formulation comprising between 1µg to 10µg of a purified intestinal cell membrane associated antigen wherein the intestinal cell membrane associated antigen is H-gal-GP and/or H11 antigen, or species specific variant, mutant or homologue thereof from a parasitic nematode for use in vaccinating a susceptible animal.

4. Use of a purified native parasitic nematode integral intestinal cell membrane antigen obtained from an infected animal wherein the intestinal cell membrane associated antigen is H-gal-GP and/or H11 antigen, or species specific variant, mutant or homologue thereof in the manufacture of a medicament for vaccinating or immunising a susceptible animal against said parasitic nematode wherein the medicament is intended to be administered in a dose of between 1µg to 10µg per animal per dose.

5. The formulation, process, or use according to any preceding claim wherein the purified antigen is in an amount of less than 5µg, 4µg, 3µg or 2µg.

6. The formulation, process, or use according to any proceeding claim wherein the parasitic nematode is *Haemonchus contortus.*

7. The formulation, process, or use according to any proceeding claim further comprising an antigen adjuvant.

8. The formulation process or use according to claim 7 wherein the adjuvant is QuilA

9. The formulation, process, method or use according to any proceeding claim further comprising an additional immogunenic formulation for vaccinating against a separate diseases or condition.

10. The formulation, process or use according to claim 9 wherein the disease or condition is a clostridial infection.

11. The formulation, process, or use according to claim 10 wherein the formulation is Covexin 8.

12. The use or process according to any of claims 4-8 wherein the susceptible animal is to be initially administered with two doses of antigen.

13. The use according to claim 12 wherein each of the said two doses is administered to the animal 2 - 8 weeks apart.

14. The formulation, process, or use according to any proceeding claim, wherein the animal to be treated is a sheep, goat, cattle or wild ruminant.

## Patentansprüche

1. Immunogene Formulierung zur Verwendung bei einer Methode zum Schutz und/oder zur Behandlung eines Lebewesens gegen eine parasitäre Nematode, wobei die immunogene Formulierung ein gereinigtes Integral-Intestinalzellenmembran-Glykoprotein-Antigen aus einer Nematode, gewonnen aus einem infizierten Lebewesen, umfasst, wobei das Intestinalzellenmembran-assozüerte Antigen H-gal-GP- und/oder H11-Antigen, oder ein Spezies-spezifischer Mutant davon, der ähnliche funktionelle Aktivität zeigt, in einer Menge zwischen 1 µg bis 10 µg ist.

2. Verfahren zur Herstellung einer immunogenen Formulierung zwecks Verwendung bei einer Methode zum Schutz und/oder zur Behandlung eines Lebewesens gegen eine parasitäre Nematode, welches Verfahren umfasst:
a) Isolieren von Nematoden aus einem infizierten Lebewesen;
b) Gewinnen von membranösem Intestinalzellenmaterial aus den Nematoden;
c) Reinigen eines Intestinalzellenmembran-assoziierten Antigens aus dem membranösen Material, wobei das Intestinalzellenmembran-assozüerte Antigen H-gal-GP- und/oder H11-Antigen ist, oder ein/-e Spezies-spezifische/-r/-s Variante, Mutant oder Homolog davon, und
Herstellen der Formulierung, umfassend zwischen 1 µg bis 10 µg des gereinigten Antigens.

3. Immunogene Formulierung, umfassend zwischen 1 µg bis 10 µg eines gereinigten Intestinalzellenmembran-assoziierten Antigens, wobei das Intestinalzellenmembranassoziierte Antigen H-gal-GP- und/oder H11-Antigen ist, oder ein/-e Speziesspezifische/-r/-s Variante, Mutant oder Homolog davon, aus einer parasitären Nematode, zwecks Verwendung beim Impfen eines suszeptiblen Lebewesens.

4. Verwendung eines gereinigten nativen Parasitnematoden-Integral-Intestinalzellenmembran-Antigens, gewonnen aus einem infizierten Lebewesen, wobei das Intestinalzellenmembran-assozüerte Antigen H-gal-GP- und/oder H11-Antigen ist, oder ein/-e Spezies-spezifische/-r/-s Variante, Mutant oder Homolog davon, bei der Herstellung eines Medikaments zur Impfung oder Immunisierung eines suszeptiblen Lebewesens gegen die parasitäre Nematode, wobei das Medikament zur Verabreichung in einer Dosis zwischen 1 µg bis 10 µg pro Lebewesen pro Dosis bestimmt ist.

5. Formulierung, Verfahren oder Verwendung nach einem der vorstehenden Ansprüche, wobei das gereinigte Antigen in einer Menge von weniger als 5 µg, 4 µg, 3 µg oder 2 µg vorliegt.

6. Formulierung, Verfahren oder Verwendung nach einem der vorstehenden Ansprüche, wobei die parasitäre Nematode *Haemonchus contortus* ist.

7. Formulierung, Verfahren oder Verwendung nach einem der vorstehenden Ansprüche, weiterhin umfassend ein Antigenadjuvans.

8. Formulierung, Verfahren oder Verwendung nach Anspruch 7, wobei das Adjuvans QuilA ist.

9. Formulierung, Verfahren, Methode oder Verwendung nach einem der vorstehenden Ansprüche, weiterhin umfassend eine zusätzliche immunogene Formulierung zur Impfung gegen eine separate Krankheit oder einen separaten Zustand.

10. Formulierung, Verfahren oder Verwendung nach Anspruch 9, wobei die Krankheit oder der Zustand eine Clostridieninfektion ist.

11. Formulierung, Verfahren oder Verwendung nach Anspruch 10, wobei die Formulierung Covexin 8 ist.

12. Verwendung oder Verfahren nach einem der Ansprüche 4 - 8, wobei dem suszeptiblen Lebewesen anfänglich zwei Dosen Antigen zu verabreichen sind.

13. Verwendung nach Anspruch 12, wobei jede der beiden Dosen dem Lebewesen in einem Abstand von 2 - 8 Wochen verabreicht wird.

14. Formulierung, Verfahren oder Verwendung nach einem der vorstehenden Ansprüche, wobei das zu behandelnde Lebewesen ein Schaf, eine Ziege, ein Rind oder ein Wildwiederkäuer ist.

## Revendications

1. Formulation immunogénique destinée à être utilisée dans une méthode pour protéger et/ou traiter un animal contre un nématode parasite, laquelle formulation immunogénique comprend un antigène glycoprotéique de membrane cellulaire intestinale entier purifié provenant d'un nématode obtenu chez un animal infecté, où l'antigène associé à la membrane cellulaire intestinale est l'antigène H-gal-GP et/ou H11, ou un mutant de ceux-ci spécifique à l'espèce et présentant une activité fonctionnelle similaire, en une quantité entre 1 µg et 10 µg.

2. Procédé de préparation d'une formulation immunogénique destinée à être utilisée dans une méthode pour protéger et/ou traiter un animal contre un nématode parasite, lequel procédé comprend:
a) l'isolement de nématodes à partir d'un animal infecté;
b) l'obtention d'un matériel membranaire cellulaire intestinal à partir desdits nématodes;
c) la purification d'un antigène associé à la membrane cellulaire intestinale à partir dudit matériel membranaire, lequel antigène associé à la membrane cellulaire intestinale est l'antigène H-gal-GP et/ou H11, ou un variant, mutant ou homologue de ceux-ci spécifique à l'espèce, et
la préparation de ladite formulation comprenant entre 1 µg et 10 µg dudit antigène purifié.

3. Formulation immunogénique comprenant entre 1 µg et 10 µg d'un antigène associé à la membrane cellulaire intestinale purifié, lequel antigène associé à la membrane cellulaire intestinale est l'antigène H-gal-GP et/ou H11, ou un variant, mutant ou homologue de ceux-ci spécifique à l'espèce, provenant d'un nématode parasite, destinée à être utilisée pour la vaccination d'un animal sensible.

4. Utilisation d'un antigène de membrane cellulaire intestinale entier de nématodes parasites natif purifié obtenu chez un animal infecté, lequel antigène associé à la membrane cellulaire intestinale est l'antigène H-gal-GP et/ou H11, ou un variant, mutant ou homologue de ceux-ci spécifique à l'espèce, dans la fabrication d'un médicament destiné à la vaccination ou à l'immunisation d'un animal sensible contre ledit nématode parasite, où le médicament est prévu pour être administré à une dose comprise entre 1 µg et 10 µg par animal par dose.

5. Formulation, procédé, ou utilisation selon n'importe quelle revendication précédente, où l'antigène purifié est en une quantité inférieure à 5 µg, 4 µg, 3 µg ou 2 µg.

6. Formulation, procédé, ou utilisation selon n'importe quelle revendication précédente, où le nématode parasite est *Haemonchus contortus.*

7. Formulation, procédé, ou utilisation selon n'importe quelle revendication précédente, comprenant en outre un adjuvant pour antigène.

8. Formulation, procédé, ou utilisation selon la revendication 7, où l'adjuvant est QuilA.

9. Formulation, procédé, méthode ou utilisation selon n'importe quelle revendication précédente, comprenant en outre une formulation immunogénique supplémentaire pour la vaccination contre une maladie ou affection distincte.

10. Formulation, procédé, ou utilisation selon la revendication 9 où la maladie ou affection est une infection à Clostridium.

11. Formulation, procédé, ou utilisation selon la revendication 10 où la formulation est Covexin 8.

12. Utilisation ou procédé selon l'importe lesquelles des revendications 4 à 8 où l'on doit administrer initialement à l'animal sensible deux doses d'antigène.

13. Utilisation selon la revendication 12 dans laquelle chacune desdites deux doses est administrée à l'animal à 2-8 semaines d'intervalle.

14. Formulation, procédé, ou utilisation selon n'importe quelle revendication précédente, où l'animal à traiter est un mouton, une chèvre, un bovin, ou un ruminant sauvage.
